# FASCICULE DE BREVET EUROPEEN

(11) **EP 0 766 510 B1**
(45) Date de publication et mention de la délivrance du brevet: **17.03.1999**
(21) Numéro de dépôt: 95926406.0
(22) Date de dépôt: 20.07.1995
(51) Int. Cl.: A01K 7/06, A61K 7/48, A61K 7/42, A61K 35/78, C12N 5/00

(54) **UTILISATION D'UN EXTRAIT DE LA PLANTE COMMIPHORA MUKUL COMME AGENT PIGMENTANT**
VERWENDUNG EINES EXTRAKTES AUS COMMIPHORA MUKUL ALS PIGMENT
USE OF A COMMIPHORA MUKUL EXTRACT AS A PIGMENTING AGENT

(30) Priorité: 22.07.1994 FR 9409092
(43) Date de publication de la demande: 09.04.1997
(73) Titulaire: LVMH RECHERCHE, 92752 Nanterre (FR)
(72) Inventeur: BONTE, Frédéric, 92400 Courbevoie (FR); BERVILLE, Régine, 92400 Becon-les-Bruyères (FR); DUMAS, Marc, 92700 Colombes (FR); MEYBECK, Alain, 92400 Courbevoie (FR)
(74) Mandataire: Portal, Gérard
(86) Numéro de dépôt international: FR9500977
(87) Numéro de publication internationale: WO9603033

(56) Documents cités:
- EP-A- 0 313 347
- EP-A- 0 513 671
- WO-A-94/13260

## Description

L'invention concerne essentiellement l'utilisation d'un extrait de la plante Commiphora mukul comme agent pigmentant ou de culture de mélanocytes ; ou pour la fabrication d'une composition cosmétique ou pharmaceutique, ou pour la culture des mélanocytes.

On sait que la plante Commiphora mukul fait partie de la famille des Burséracées. Le Commiphora mukul est une plante originaire de l'Inde très utilisée en médecine traditionnelle de l'Inde, en médecine Ayurvédique. En particulier dans ces applications, on utilise une résine produite par Commiphora mukul appelée également Guggul. Dans ce traitement ayurvédique, on connaissait le traitement de l'obésité et des désordres lipidiques ainsi que des maladies rhumatismales.

Il est à noter que le terme "guggul" désigne à la fois la plante et la résine qu'elle produit. Par ailleurs, cette plante est un petit arbre ou un arbuste de 1,2 à 1,8 m de hauteur qui pousse essentiellement en Inde et l'incision de la plante permet de récolter la gomme-résine de manière courante.

Récemment, les documents US-A-4 847 071, US-A-4 847 069, US-A-4 946 671 et US-A-4 954 332, ayant tous pour titulaire Procter et Gamble, décrivent des compositions topiques pour la protection contre les radiations UV, contenant des absorbeurs de radicaux libres et un agent anti-inflammatoire. Parmi les nombreux agents anti-inflammatoires cités se trouve le Guggul ou extrait de Guggul.

D'autre part, il est également connu par le document EP-A-513 671 INDENA des compositions contenant un extrait lipophile total de la plante Commiphora mukul en particulier obtenu à partir de la résine d'écorce de Commiphora mukul comme ingrédient actif. Cet extrait a une forte proportion en Guggulstérones. Cette composition est décrite comme présentant une activité anti-inflammatoire, immunomodulatrice ou anti-androgène pour le traitement de l'acné et de l'hypertrophie bénigne de la prostate.

Il a été maintenant découvert de manière surprenante et inattendue que les extraits de la plante Commiphora mukul présentent une activité pigmentante et peuvent ainsi être utilisés comme agent pigmentant ainsi que pour la fabrication de composition cosmétique ou pharmaceutique, notamment dermatologique, à activité pigmentante; ou présentent encore une activité facilitant l'acquisition par les mélanocytes de leurs caractères de différenciation et peuvent ainsi être utilisés dans le cadre de la culture de mélanocytes, en particulier sous forme de coculture avec des kératinocytes pour la préparation d'épidermes artificiels. Plus particulièrement, il a pu être observé que les extraits de la plante Commiphora mukul favorisent la formation de dendrites dont il est bien connu qu'ils participent au processus de pigmentation de la peau ou des cheveux. A cet égard, il est rappelé que lorsque le mélanocyte est activé, et qu'il produit de la mélanine sous forme de mélanosomes, la forme du mélanocyte se modifie de manière à former des sortes de "tentacules", appelées généralement "dendrites", l'aspect du mélanocyte est dit "rhizomique". Les pigments mélaniques sont alors acheminés par les dendrites vers les couches supérieures de l'épiderme. L'aspect "rhizomique" est donc un excellent critère de mise en évidence de l'activité de la mélanogénèse. Par contre, au repos, les mélanocytes perdent cet aspect pour reprendre une forme plus régulière. Différentes publications détaillées ont été faites à ce sujet, en particulier par J.P. LACOUR et al. (J. Cell Physiol. (1992) 151 287-299), et dans l'ouvrage Dermatology in General Medicine, Ed. Mc Graw Hill, (1987) pages 224-251.

Ainsi, la présente invention a pour but de résoudre le nouveau problème technique consistant en la fourniture de nouveaux agents pigmentants, de nouvelles compositions de culture de mélanocytes, en particulier des mélanocytes normaux, ou de nouvelles compositions cosmétiques ou pharmaceutiques, notamment dermatologiques, à activité pigmentante notamment pour favoriser la pigmentation de la peau ou des phanères, en particulier des cheveux, ou prévenir l'apparition des cheveux gris.

Par le terme "phanères", on entend plus particulièrement les cheveux, les cils et les sourcils.

Ce problème technique a été résolu par la présente invention par la découverte inattendue que les extraits de la plante Commiphora mukul présentaient ces activités.

Ainsi, selon un premier aspect, la présente invention concerne l'utilisation d'au moins un extrait de la plante Commiphora mukul, comme agent cosmétique favorisant la pigmentation de la peau ou des phanères, en particulier des cheveux, ou à prévenir l'apparition des cheveux gris.

Selon un deuxième aspect, la présente invention concerne l'utilisation d'au moins un extrait de la plante Commiphora mukul pour la préparation d'une composition pharmaceutique destinée à favoriser la pigmentation de la peau ou des phanères, en particulier des cheveux, ou à prévenir l'apparition des cheveux gris.

Selon un troisième aspect, la présente invention concerne l'utilisation d'au moins un extrait de la plante Commiphora mukul, comme agent de culture de mélanocytes, en particulier de mélanocytes normaux, de préférence de mélanocytes humains normaux.

Selon un mode de réalisation avantageux, l'extrait de la plante Commiphora mukul est un extrait de la résine de cette plante, encore appelée Guggul.

Selon encore un mode de réalisation particulier, l'extrait végétal précité est un extrait lipophile de résine de la plante Commiphora mukul.

Selon un mode de réalisation avantageux, l'extrait lipophile est obtenu par extraction au moyen d'un solvant ou d'un mélange de solvants caractérisé par un paramètre de polarité p' de valeur inférieure à 5,5. Il est rappelé que le paramètre de polarité p' est défini dans la littérature, en particulier par la publication de L.R. SNYDER (J. Chromatogr. Sci., (1978) 16, 223-234), ou celle de V.R. MEYER (dans Practical high Performance Liquid Chromatography, John Wiley Ed., New-York, 1988, pages 120-121). Parmi ces solvants, on utilisera bien entendu de préférence ceux ne présentant pas de toxicité notable, en particulier le méthanol, l'éthanol et l'acétate d'éthyle, ayant pour valeur du paramètre p' respectivement : 5,1, 4,3 et 4,4.

Selon un autre mode de réalisation avantageux de l'invention, la concentration en extrait végétal est comprise entre 0,001 % et 1 %, de préférence entre 0,01 % et 0,1 %, en pourcentage en poids de la composition finale cette composition contenant éventuellement un excipient cosmétiquement ou pharmaceutiquement acceptable.

Selon un autre mode de réalisation particulièrement avantageux, la composition précitée contient des phases lamellaires lipidiques hydratées ou des liposomes incorporant ou non l'extrait végétal précité.

Selon une variante de réalisation particulière, l'extrait végétal est présent dans la phase lipidique des phases lamellaires lipidiques hydratées ou des liposomes.

Selon un autre mode de réalisation particulier, la composition précitée est formulée sous une forme permettant une application topique, en particulier sous forme de crème, de gel ou de lotion destinée à l'application sur la peau ou sur les cheveux. Ainsi, divers excipients bien connus à l'homme de l'art peuvent être utilisés pour cette formulation.

Selon un quatrième aspect, la présente invention fournit encore un procédé de fabrication d'une composition cosmétique ou pharmaceutique, notamment dermatologique, destiné en particulier à favoriser la pigmentation de la peau ou des phanères, notamment des cheveux, caractérisé en ce qu'il comprend l'incorporation d'au moins un extrait végétal de la plante Commiphora mukul dans un excipient, véhicule ou support cosmétiquement ou pharmaceutiquement acceptable.

Selon un cinquième aspect, la présente invention fournit encore un procédé de culture de mélanocytes, notamment de mélanocytes normaux, de préférence des mélanocytes humains normaux, en vue de leur faire acquérir des caractères de différenciation, en particulier de maturation, convenables pour une utilisation ultérieure, caractérisé en ce qu'il comprend la culture desdits mélanocytes dans un milieu de culture comprenant une quantité efficace d'au moins un extrait de la plante Commiphora mukul, en particulier tel que précédemment défini, pendant une période de temps suffisante pour obtenir une différenciation des mélanocytes.

Selon un mode de réalisation particulier, les mélanocytes sont cocultivés avec des kératinocytes afin de préparer des épidermes artificiels. Les épidermes artificiels obtenus par cette co-culture sont traités par des extraits de Commiphora mukul selon l'invention, soit en cours de co-culture, soit après formation complète de ces épidermes, dans un délai approprié avant leur utilisation. Grâce à ce traitement, les épidermes artificiels peuvent acquérir leur fonctionnalité, en particulier celle de la pigmentation. Ces épidermes artificiels ayant acquis une pigmentation similaire à celle de la peau naturelle peuvent être utilisés, notamment à des fins de diagnostic, en particulier, pour tester des produits cosmétiques ou pharmaceutiques protecteurs ou non du rayonnement UV, ou tester des produits de maquillage. Ils peuvent également être utilisés pour réaliser des greffes d'épiderme in vivo.

La présente invention permet de mettre en oeuvre un procédé de traitement, non revendiqué en lui-même, consistant en des greffes de mélanocytes chez un mammifère, de préférence chez l'homme, caractérisé en ce que l'on greffe des mélanocytes normaux compatibles avec le mammifère concerné par la greffe, notamment des mélanocytes humains normaux chez l'homme, qui ont subi une différenciation par culture en présence d'une quantité efficace d'au moins un extrait de la plante Commiphora mukul, en particulier tel que précédemment défini, pour obtenir une différenciation des mélanocytes.

Selon une variante de réalisation, cette culture a lieu en présence de 0,0001 à 0,1 % en poids d'extrait de la plante Commiphora mukul, par rapport au poids total du milieu de culture.

Selon un sixième aspect, l'invention couvre encore un milieu de culture de mélanocytes, notamment de mélanocytes normaux, de préférence des mélanocytes humains normaux, caractérisé en ce qu'il comprend une quantité efficace pour obtenir une différenciation des mélanocytes d'au moins un extrait de la plante Commiphora mukul, tel que précédemment défini.

On comprend ainsi que l'invention permet de résoudre le nouveau problème technique précédemment énoncé ainsi que d'autres problèmes techniques qui apparaîtront clairement à l'homme de l'art à partir de la description prise dans son ensemble.

D'autres buts, caractéristiques et avantages de l'invention apparaîtront clairement à l'homme de l'art à partir de la description suivante faite en référence à plusieurs modes de réalisation actuellement préférés de l'invention, ainsi qu'aux tests d'activité qui suivent. Dans la description et les revendications, tous les pourcentages sont donnés en poids, sauf indication contraire.

### Exemple 1

### Préparation d'un extrait végétal lipophile de la résine de Commiphora mukul

On extrait 1 kg de résine de Commiphora mukul séchée et finement broyée sous agitation avec 10 l d'acétate d'éthyle tout d'abord à la température ambiante, ensuite sous reflux doux. Après filtration de la matière insoluble, le résidu est extrait à nouveau avec 10 l du même solvant dans les mêmes conditions. Les extraits recueillis sont traités avec 0,05 kg de charbon actif et, après 3 h sous reflux doux, on filtre. La solution claire jaune brillante est concentrée sous vide à 40°C jusqu'à l'obtention d'une forme pâteuse qui présente la forme d'un miel très visqueux et le résidu est dilué avec 5 l d'éthanol. La solution éthanolique est filtrée et concentrée sous vide. On obtient ainsi environ 0,42 kg d'un matériau semifluide de couleur ambre constituant un extrait selon la présente invention, dénommé I₁.

### Exemple 2

### Détermination de l'activité pigmentante d'un extrait de la plante Commiphora mukul. par le test mettant en évidence la formation de dendrites sur des mélanocytes humains normaux en culture

On effectue sur une culture de mélanocytes humains normaux un traitement par un extrait selon l'invention à différentes concentrations, puis on étudie la morphologie des mélanocytes par observation en microscopie photonique, en particulier pour apprécier la dendricité des cellules.

### a) Culture des mélanocytes

Des mélanocytes humains normaux sont obtenus à partir d'un fragment de peau humaine saine provenant de chirurgie plastique. On prépare une suspension de cellules épidemiques à partir de l'épiderme séparé du derme par incubation une nuit à 4°C dans une solution de trypsine à 0,25 %. Cette suspension de cellules épidermiques est ensuite cultivée dans un milieu inhibant la croissance kératinocytaire, tel que décrit dans les publications de A. LERNER et al. (J. Invest. Dermatol., (1987) 89 219-24) ou de B. GILCHREST (Brit. J. Dermatol. (1986) 115 (suppl. 31) 17-23), de telle sorte que l'on obtienne une population de mélanocytes.

Les mélanocytes ainsi obtenus sont cultivés de manière classique en microplaques dans un milieu E199 disponible dans le commerce chez GIBCO (France), supplémenté à 10% en poids avec du sérum de veau foetal, et supplémenté en outre avec :

| | |
|---|---|
| - transferrine | 10 µg/ml |
| - insuline | 10 µg/ml |
| - hydrocortisone | 0,4 µg/ml |
| - EGF (facteur de croissance épidermique) | 10 ng/ml |
| - généticine | 100 µg/ml |

Chaque microplaque contient 96 puits. On ensemence 15 000 mélanocytes humains normaux par puits dans le milieu de culture précité, et on les laisse adhérer à ces puits pendant un temps d'incubation de 72 h à 37°C.

Après cette durée d'incubation, le milieu de culture est retiré, et remplacé par un milieu identique contenant les produits à tester aux différentes concentrations, à l'exception des cultures témoin pour lesquels le milieu de remplacement ne contient aucun autre produit. Le traitement des cultures s'effectue sur 9 jours, avec renouvellement identique du milieu toutes les 48 h, avec au total trois renouvellements.

72 h après le dernier renouvellement, on mesure la dendricité des mélanocytes.

### b) Les produits testés

Le produit I₁ obtenu à l'exemple 1 est dissous dans du diméthyl-sulfoxyde (DMSO) à 25 mg/ml pour former une solution mère. Cette solution mère est ensuite utilisée pour fournir diverses concentrations de produits à tester à savoir 1 µg/ml ; 2,5 µg/ml; 10 µg/ml et 25 µg/ml en concentration finale.

### c) Etude de la dendricité

Pour étudier la dendricité, on réalise les observations au microscope optique disponible dans le commerce chez LEITZ, en observant les puits et en comptant les mélanocytes selon leur forme dans des champs pris au hasard. Dans ce cadre, on compte les cellules sans dendrite, et les cellules avec un ou plusieurs dendrites.

L'étude statistique est effectuée par un test bien connu à l'homme de l'art dit de χ² (Ki²), qui compare les différences de répartition des populations.

On obtient les résultats répertoriés au tableau I suivant :

**TABLEAU I**

| Résultat de mesure de dendricité sur des mélanocytes humains normaux en culture | | | | |
|---|---|---|---|---|
| Produit testé | Mélanocytes non dendritiques | | Mélanocytes dendritiques | |
| | Nombre | Pourcent | Nombre | Pourcent |
| Témoin DMSO | 69 | 46,3 | 80 | 53,7 |
| Ex. 1 : 1 µg/ml | 68 | 35,4 | 124 | 64,6 |
| Ex. 1 : 2,5 µg/ ml | 67 | 32,4 | 140 | 67,6 |
| Ex. 1 : 10 µg/ml | 32 | 14,7 | 185 | 85,3 |
| Ex. 1 : 25 µg/ml | 7 | 6,2 | 106 | 93,8 |

On peut clairement constater, à partir des résultats de mesure de dendricité répertoriés au tableau I, que l'application du produit selon l'invention contenant un extrait de la plante Commiphora mukul aboutit d une augmentation de 20 % à environ 100 % du nombre de mélanocytes dendritiques, en fonction de la concentration appliquée, ce qui est tout à fait remarquable.

D'autre part, il est à noter que l'étude statistique montre une variation de statistique significative à 1 %.

Dans ces conditions, étant donné que le phénomène de dendricité intervient dans le processus d'activation des mélanocytes et du transport de la mélanine formée vers les kératinocytes, une augmentation du nombre de mélanocytes dendritiques constitue une mise en évidence de l'efficacité du produit testé sur l'activation des mélanocytes, ainsi que d'une stimulation de l'activité pigmentante, comme cela a déja' été exposé plus haut.

### Exemple 3

### Test d'évaluation de l'activité sur la mélanogénèse

Dans le cadre de ce test, on effectue sur des mélanocytes humains normaux cultivés un dosage de mélanine que l'on rapporte à un taux de protéine pour tenir compte des variations possibles du nombre des cellules de mélanocytes. La mélanine est évaluée suivant une méthode classique par spectrométrie à 405 nm. La culture des mélanocytes est effectuée selon la méthode décrite à l'exemple 2, si ce n'est que l'ensemencement de la culture est réalisé à raison de 40 000 cellules par puits.

Pour réaliser ce dosage de la mélanine, on prélève le milieu dans les micropuits par aspiration. Les micropuits sont ensuite rincés avec un tampon PBS.

Les cellules de mélanocytes restent accrochées aux parois des puits.

Ensuite, on ajoute 120 µl de soude 0,5 M à chaque puits. On réalise une agitation pendant 15 min à la température ambiante.

On recueille la solution de soude et on procède à une mesure de la densité optique à 405 nm dans un appareil de mesure de la densité optique disponible dans le commerce chez Perkin Elmer. Cette mesure de la densité optique est comparée à une solution témoin constituée par la solution de soude 0,5 M pure.

Les valeurs de densité optique ainsi obtenues sont converties en microgrammes de mélanine par rapport à une courbe étalon obtenue avec de la mélanine synthétique disponible dans le commerce auprès de la société SIGMA, FRANCE, référence M 8631.

Le dosage des protéines effectué est basé sur une technique mettant en oeuvre l'acide bicinchoninique. Cette technique est disponible dans le commerce chez SIGMA - FRANCE -, sous la forme du Kit BCA-1, réf. B9643. L'étalonnage du dosage des protéines est effectué avec de la sérum-albumine bovine. Les résultats obtenus sont répertoriés au tableau II ci-après.

**TABLEAU II**

| Dosage de mélanine sur des mélanocytes humains normaux cultives selon l'exemple 2 | | |
|---|---|---|
| Concentration produits testés | Mélanine µg Protéine µg | Augmentation sur la mélanogénèse |
| Témoin | 1,6 x 10⁻² | 0 |
| 1 µg/ml | 1,9 x 10⁻² | 19% |
| 2,5 | 2,4 x 10⁻² | 50% |
| 10 | 2,7 x 10⁻² | 69% |
| 25 | 2,2 x 10⁻² | 37% |

On a reporté en deuxième colonne du tableau II la quantité de mélanine formée rapportée à la quantité de protéine contenue dans la culture, en fonction de la concentration des produits testés. L'activité sur la mélanogénèse, figurant en troisième colonne du tableau, a été calculée par comparaison avec le taux de mélanine produite dans la culture témoin.

Il apparaît très clairement à partir de ces résultats que les extraits selon l'invention présentent une importante activité de stimulation de la production du pigment mélanique par les mélanocytes.

Ainsi, ces résultats, rapprochés de ceux de l'exemple 2, mettant en évidence l'amélioration du transfert des pigments mélaniques vers les kératinocytes, démontrent que les extraits selon l'invention favorisent une augmentation de la pigmentation de l'épiderme et des phanères, tels que les cheveux.

Divers exemples de formulation de compositions cosmétiques ou pharmaceutiques, notamment dermatologiques sont donnés ci-après.

### Exemples d'application

### Exemple 4

### . Lotion alcoolique capillaire

| | |
|---|---|
| Extrait I₁ selon l'exemple 1 : | 0,05 % |
| Huile de ricin hydrogénée PEG-40 : | 0,5 % |
| Ethanol absolu : | 10% |
| Eau déminéralisé qsp : | 100 ml |

### Exemple 5

### . Gel alcoolique

| | |
|---|---|
| Extrait I₁ selon l'exemple 1 : | 0,02 |
| Huile de ricin hydrogénée PEG-40 : | 0,5 |
| Ethanol absolu : | 15 |
| Carbopol 980® : | 2 |
| Eau + Conservateur qsp : | 100 g |

en application locale.

### Exemple 6

### . Emulsion bronzante

### 1)Composant A :

| | |
|---|---|
| PEG-6 : | 7% |
| Alcool cétylique : | 3% |
| Huile minérale : | 25% |
| Extrait I₁ selon l'exemple 1 : | 0,2% |
| Benzoate d'alkyle C₁₂₋₁₅ : | 1,8 % |

### 2)Composant B :

| | |
|---|---|
| Conservateur : | 6,5% |
| Eau déminéralisée : | 45,2% |
| NaOH à 1% : | 6,5% |

### 3)Composant C:

| | |
|---|---|
| Gel de Carbopol 980® à 1,5 % : | 12,00. |

Mélanger B et C, homogénéiser.
Chauffer séparément A ; B + C à 70°C.
Emulsionner au Raynerie le composant A avec les composants B + C à 70°C pendant 1 h 30.

L'émulsion s'emploie pour corriger les défauts de pigmentation cutanée en traitement local 2 fois par jour pendant 3 semaines.

### Exemple 7

### . Poudre compacte colorée

### Phase A:

| | |
|---|---|
| Myristate de magnésium : | 4 |
| Orgasol 2002 UD Nat Cos® (Atochem) : | 14 |
| Talc OOC de Luzenac : | 40,3 |
| Parabènes : | 0,2 |

### Phase B :

| | |
|---|---|
| Oxyde de fer noir W 9814 : | 0,5 |
| Oxyde de fer brun : | 8 |
| Oxyde de titane UFTR : | 5 |
| Pigments nacrés : | 20 |
| Golden bronze (Mecul) : 4 | |
| Lustra pearl glimmer (Mallinkrodt) : 16) | |

### Phase C :

| | |
|---|---|
| Isostéarate d'isostearyle : | 2,5 |
| PCL liquide (2/066210) : | 4 |
| Huile d'abricot : | 1,45 |
| Extrait I₁ selon l'exemple 1, dissout dans 90 % de benzoate d'alkyle C₁₂₋₁₅ : | 0,05 |

### Mode opératoire :

. Peser A : Mélanger intimement.
. Peser B : Mélanger intimement.
Introduire C petit à petit tout en mélangeant.
Compacter.

La poudre obtenue s'applique en maquillage soin et aide la peau à acquérir une teinte bronzée idéale rapidement.

### Exemple 8

### . Milieu de culture pour mélanocytes humains normaux

| | |
|---|---|
| Extrait selon l'exemple 1 | 0,005 % |
| Sérum de veau foetal | 10% |
| Transferrine | 10 µg/ml |
| Hydrocortisone | 0,35 µg/ml |
| Généticine | 100 µg/ml |
| L-glutamine | 2 mM |
| E.G..F. (facteur de croissance épidermique) | 5 ng/ml |
| Milieu E 199 | qsp 100 ml |

## Revendications

1. Utilisation d'au moins un extrait de la plante Commiphora mukul comme agent cosmétique favorisant la pigmentation de la peau ou des phanères, en particulier des cheveux, ou prévenant l'apparition des cheveux gris.

2. Utilisation d'au moins un extrait de la plante Commiphora mukul pour la préparation d'une composition pharmaceutique, notamment dermatologique, destinée à favoriser la pigmentation de la peau ou des phanères, en particulier des cheveux, ou à prévenir l'apparition des cheveux gris.

3. Utilisation d'au moins un extrait de la plante Commiphora mukul comme agent de culture de mélanocytes, en particulier de mélanocytes normaux, de préférence des mélanocytes humains normaux.

4. Utilisation selon l'une des revendications 1 à 3, caractérisée en ce que l'extrait de la plante Commiphora mukul est un extrait de la résine de cette plante, encore appelée Guggul.

5. Utilisation selon l'une des revendications 1 à 4, caractérisée en ce que l'extrait végétal précité est un extrait lipophile de résine de la plante Commiphora mukul.

6. Utilisation selon la revendication 5, caractérisée en ce que l'extrait lipophile précité est obtenu par extraction au moyen d'un solvant ou d'un mélange de solvants caractérisé par un paramètre de polarité p' de valeur inférieure à 5,5.

7. Utilisation selon la revendication 6, caractérisée en ce que le solvant ne présente pas de toxicité notable, en particulier l'éthanol, le méthanol et l'acétate d'éthyle.

8. Utilisation selon l'une des revendications précédentes, caractérisée en ce que la concentration en extrait végétal est comprise entre 0,001 % et 1 %, de préférence entre 0,01 % et 0,1 %, en pourcentage en poids de la composition finale; cette composition contenant éventuellement un excipient cosmétiquement ou pharmaceutiquement acceptable.

9. Utilisation selon l'une des revendications 1, 2, 4 à 8, caractérisée en ce que ladite composition contient des phases lamellaires lipidiques hydratées ou des liposomes incorporant ou non l'extrait végétal précité.

10. Utilisation selon la revendication 9, caractérisèe en ce que l'extrait végétal est présent dans la phase lipidique des phases lamellaires lipidiques hydratées ou des liposomes.

11. Utilisation selon l'une des revendications 1, 2, 4 à 10, caractérisée en ce que ladite composition est formulée sous une forme permettant une application topique, en particulier sous forme de crème, de gel ou de lotion destiné à l'application sur la peau ou sur les cheveux.

12. Procédé de fabrication d'une composition cosmétique ou pharmaceutique, notamment dermatologique, destinée en particulier à favoriser la pigmentation de la peau ou des phanères, notamment des cheveux, caractérisé en ce qu'il comprend l'incorporation d'au moins un extrait végétal de la plante Commiphora mukul tel que défini à l'une quelconque des revendications 1, 2, 4 à 11, dans un excipient, véhicule ou support cosmétiquement ou pharmaceutiquement acceptable.

13. Procédé de culture de mélanocytes, notamment de mélanocytes normaux, de préférence des mélanocytes humains normaux, en vue de leur faire acquérir des caractères de différenciation, en particulier de maturation, convenables pour une utilisation ultérieure, caractérisé en ce qu'il comprend la culture desdits mélanocytes dans un milieu de culture comprenant une quantité efficace d'au moins un extrait de la plante Commiphora mukul, en particulier tel que défini aux revendications 3 à 7, pendant une période de temps suffisante pour obtenir une différenciation des mélanocytes.

14. Procédé selon la revendication 13, caractérisé en ce que les mélanocytes sont co-cultivés avec des kératinocytes afin de préparer des épidermes artificiels, destinés notamment à des fins de diagnostic, à tester des produits cosmétiques ou pharmaceutiques, protecteurs ou non du rayonnement UV, à tester des produits de maquillage, ou encore à réaliser des greffes d'épiderme in vivo.

15. Procédé selon la revendication 13 ou 14, caractérisé en ce que la culture a lieu en présence de 0,0001 à 0,1 % en poids d'extrait de la plante Commiphora mukul, par rapport au poids total du milieu de culture.

16. Milieu de culture de mélanocytes, notamment de mélanocytes normaux, de préférence des mélanocytes humains normaux, caractérisé en ce qu'il comprend une quantité efficace pour obtenir une différenciation des mélanocytes d'au moins un extrait de la plante Commiphora mukul, en particulier tel que défini aux revendications 3 à 7, 13 à 15.

17. Procédé cosmétique de pigmentation de la peau ou des phanères, en particulier des cheveux, de prévention de l'apparition des cheveux gris, caractérisé en ce qu' on applique sur les zones à pigmenter de la peau ou des phanères une quantité efficace d'au moins un extrait de la plante Commiphora mukul, en particulier tel que défini à l'une quelconque des revendications 1, 4 à 11.

## Claims

1. Use of at least one *Commiphora mukul* plant extract as a cosmetic agent promoting the pigmentation of the skin or the integuments, in particular the hair, or for preventing the appearance of grey hair.

2. Use of at least one *Commiphora mukul* plant extract for the preparation of a pharmaceutical composition, notably a dermatological composition, intended for promoting the pigmentation of the skin or the integuments, in particular the hair, or for preventing the appearance of grey hair.

3. Use of at least one *Commiphora mukul* plant extract as a melanocyte culture agent, in particular normal melanocytes, preferably normal human melanocytes.

4. Use according to one of claims 1 to 3, characterised in that the *Commiphora mukul* plant extract is an extract of the resin of this plant, also called Guggul.

5. Use according to one of claims 1 to 4, characterised in that the aforementioned plant extract is a lipophilic extract of resin of the *Commiphora mukul* plant.

6. Use according to claim 5, characterised in that the aforementioned lipophilic extract is obtained by extraction by means of a solvent or a mixture of solvents characterised by a polarity parameter p' whose value is less than 5.5.

7. Use according to claim 6, characterised in that the solvent does not possess any notable toxicity, in particular ethanol, methanol and ethyl acetate.

8. Use according to one of the preceding claims, characterised in that the concentration of plant extract is between 0.001 % and 1%, preferably between 0.01% and 0.1%, in percentage by weight of the final composition; this composition optionally containing a cosmetically or pharmaceutically acceptable excipient.

9. Use according to one of claims 1, 2, 4 to 8, characterised in that said composition contains hydrated lipidic lamellar phases or liposomes incorporating or not the aforementioned plant extract.

10. Use according to claim 9, characterised in that the plant extract is present in the lipidic phase of the hydrated lipidic lamellar phases or liposomes.

11. Use according to one of claims 1, 2, 4 to 10, characterised in that said composition is formulated in a form which allows a topical application, in particular in the form of a cream, a gel or a lotion intended for the application on the skin or the hair.

12. A process for the manufacture of a cosmetic or pharmaceutical composition, notably a dermatological composition, intended in particular for promoting the pigmentation of the skin or the integuments, notably the hair, characterised in that it comprises the incorporation of at least one plant extract of the *Commiphora mukul* plant as defined in any one of claims 1, 2, 4 to 11, in a cosmetically or pharmaceutically acceptable excipient, vehicle or support.

13. A process for the culture of melanocytes, notably of normal melanocytes, preferably normal human melanocytes, with the view of making them acquire differentiation features, in particular maturation, suitable for a subsequent use, characterised in that said process comprises the culture of said melanocytes in a culture medium comprising an effective amount of at least one *Commiphora mukul* plant extract, in particular as defined in claims 3 to 7, for a period of time sufficient for obtaining a differentiation of the melanocytes.

14. The process according to claim 13, characterised in that the melanocytes are co-cultivated with keratinocytes so as to prepare artificial epidermes, intended notably for diagnostic purposes, for testing pharmaceutical or cosmetic products which are protective or not against UV rays, for testing makeup products, or even for carrying out epidermis grafts *in vivo.*

15. The process according to claim 13 or 14, characterised in that the culture takes place in the presence of 0.0001 to 0.1 % by weight of *Commiphora mukul* plant extract with respect to the total weight of the culture medium.

16. A melanocyte culture medium, notably of normal melanocytes, preferably normal human melanocytes, characterised in that it comprises an effective amount of at least one extract of the *Commiphora mukul* plant, in particular an extract of the *Commiphora mukul* plant as defined in claims 3 to 7, 13 to 15, for obtaining a differentiation of the melanocytes.

17. A cosmetic process of pigmentation of the skin or the integuments, in particular the hair, of prevention of the appearance of grey hair, characterised in that an effective amount of at least one extract of the *Commiphora mukul* plant, in particular an extract of the *Commiphora mukul* plant as defined in any one of claims 1, 4 to 11, is applied on the areas to be pigmented of the skin or the integuments.

## Patentansprüche

1. Verwendung zumindest eines Extrakts der Pflanze Commiphora mukul als kosmetisches Mittel zur Förderung der Pigmentation der Haut oder Anhangsgebilde, insbesondere der Haare, oder zur Prävention des Auftretens grauer Haare.

2. Verwendung zumindest eines Extrakts der Pflanze Commiphora mukul bei der Herstellung einer pharmazeutischen, insbesondere dermatologischen Zusammensetzung zur Förderung der Pigmentation der Haut oder Anhangsgebilde, insbesondere der Haare, oder zur Prävention des Auftretens grauer Haare.

3. Verwendung zumindest eines Extrakts der Pflanze Commiphora mukul als Kulturmittel für Melanocyten, insbesondere normale Melanocyten, vorzugsweise normale humane Melanocyten.

4. Verwendung nach einem der Ansprüche 1 bis 3, dadurch gekennzeichnet, daß der Extrakt der Pflanze Commiphora mukul ein Harzextrakt dieser Pflanze ist, der auch als Guggul bezeichnet wird.

5. Verwendung nach einem der Ansprüche 1 bis 4, dadurch gekennzeichnet, daß der Pflanzenextrakt ein lipophiler Harzextrakt der Pflanze Commiphora mukul ist.

6. Verwendung nach Anspruch 5, dadurch gekennzeichnet, daß der lipophile Extrakt durch Extraktion mit einem Lösungsmittel oder einer Lösungsmittelmischung erhalten wird, das bzw. die durch einen Polaritätsparameter p' mit einem Wert von weniger als 5,5 gekennzeichnet ist.

7. Verwendung nach Anspruch 6, dadurch gekennzeichnet, daß das Lösungsmittel keinerlei merkbare Toxizität aufweist, insbesondere Ethanol, Methanol und Ethylacetat.

8. Verwendung nach einem der vorhergehenden Ansprüche, dadurch gekennzeichnet, daß die Konzentration des Pflanzenextrakts zwischen 0,001 % und 1 %, vorzugsweise zwischen 0,01 % und 0,1 %, bezogen auf die Masse der Endzusammensetzung, beträgt, wobei diese Zusammensetzung gegebenenfalls einen kosmetisch oder pharmazeutisch annehmbaren Exzipienten enthält.

9. Verwendung nach einem der Ansprüche 1, 2, 4 bis 8, dadurch gekennzeichnet, daß die Zusammensetzung hydratisierte lamellare Lipidphasen oder Liposomen, die gegebenenfalls den Pflanzenextrakt einschließen, enthält.

10. Verwendung nach Anspruch 9, dadurch gekennzeichnet, daß der Pflanzenextrakt in der Lipidphase von hydratisierten lamellaren Lipidphasen oder Liposomen vorliegt.

11. Verwendung nach einem der Ansprüche 1, 2, 4 bis 10, dadurch gekennzeichnet, daß die Zusammensetzung in einer Form formuliert ist, die eine topische Verwendung, insbesondere in Form einer Creme, eines Gels oder einer Lotion zum Aufbringen auf der Haut oder auf den Haaren, ermöglicht.

12. Verfahren zur Herstellung einer kosmetischen oder pharmazeutischen, insbesondere dermatologischen Zusammensetzung, insbesondere zur Förderung der Pigmentation der Haut oder Anhangsgebilde, insbesondere der Haare, dadurch gekennzeichnet, daß es den Einschuß zumindest eines Pflanzenextrakts der Pflanze Commiphora mukul, wie in einem der Ansprüche 1, 2, 4 bis 11 definiert, in einen kosmetisch oder pharmazeutisch annehmbaren Exzipienten, in ein Vehikel oder in einen Träger umfaßt.

13. Verfahren zum Kultivieren von Melanocyten, insbesondere normalen Melanocyten, vorzugsweise normalen humanen Melanocyten, um ihnen für eine spätere Verwendung geeignete Charakteristika der Differenzierung, insbesondere Reifung, zu verleihen, dadurch gekennzeichnet, daß es die Kultur der Melanocyten in einem Kulturmedium, das eine wirksame Menge zumindest eines Extrakts der Pflanze Commiphora mukul, insbesondere wie in den Ansprüchen 3 bis 7 definiert, umfaßt, während eines ausreichenden Zeitraums umfaßt, um eine Differenzierung der Melanocyten zu erhalten.

14. Verfahren nach Anspruch 13, dadurch gekennzeichnet, daß die Melanocyten mit Keratinocyten co-kultiviert werden, um künstliche Epidermis herzustellen, die insbesondere für diagnostische Zwecke, zum Testen kosmetischer oder pharmazeutischer, gegebenenfalls gegen UV-Strahlung schützender Produkte, zum Testen von Schminkprodukten oder auch zur Durchführung von Epidermistransplantationen in vivo bestimmt ist.

15. Verfahren nach Anspruch 13 oder 14, dadurch gekennzeichnet, daß die Kultur in Anwesenheit von 0,0001 bis 0,1 Masse-% Extrakt der Pflanze Commiphora mukul, bezogen auf die Gesamtmasse des Kulturmediums, stattfindet.

16. Medium zum Kultivieren von Melanocyten, insbesondere normalen Melanocyten, vorzugsweise normalen humanen Melanocyten, dadurch gekennzeichnet, daß es eine wirksame Menge, um eine Differenzierung von Melanocyten zu erhalten, zumindest eines Extrakts der Pflanze Commiphora mukul, insbesondere wie in den Ansprüchen 3 bis 7, 13 bis 15 definiert, umfaßt.

17. Kosmetisches Verfahren zur Pigmentation der Haut oder der Anhangsgebilde, insbesondere der Haare, zur Prävention des Auftretens grauer Haare, dadurch gekennzeichnet, daß auf die zu pigmentierenden Zonen der Haut oder Anhangsgebilde eine wirksame Menge zumindest eines Extrakts der Pflanze Commiphora mukul, insbesondere wie in einem der Ansprüche 1, 4 bis 11 definiert, aufgebracht wird.
